# EUROPEAN PATENT APPLICATION

(11) **EP 1 645 250 A2**
(43) Date of publication of application: **12.04.2006**
(21) Application number: 05018381.3
(22) Date of filing: 24.08.2005
(51) Int. Cl.: A61F 5/01, A43B 13/36

(54) **Interchangeable sole for walkers**

(30) Priority: 25.08.2004 IT VR20040131
(71) Applicant: F.G.P. SRL, 37062 Dossobuono (IT)
(72) Inventor: Turrini, Alberto, 37062 Castel D'Azzano (VR) (IT); Ferrigolo, Moreno, 37062 Dossobuono (VR) (IT)
(74) Representative: Petraz, Gilberto Luigi

(57) **Abstract**

A sole (16) which can be applied in particular to plantar type walkers (10) for the foot and/or the ankle or for the lower part of the leg, in which the walker presents a casing (11) in which the foot is inserted; the sole (16) is the removable type and the casing of the walker can be fixed to the removable sole (16) by means of non-permanent bi-adhesive material.

## Description

### TECHNICAL FIELD

This invention concerns an interchangeable sole particularly designed for application on plantar walkers for the foot and/or the ankle.

More specifically, this invention refers to a sole that can be fitted on walkers or arch supports, in particular those with a structure made from rigid material, such as plastic or lightweight metal alloy, with appropriate joints at the level of the ankle, with a frame that can be used following bruising and/or sprain type injuries involving the foot, the heel and/or the ankle.

The sole according to the invention is fitted on the sole of the walker by means of temporary adhesive systems, such as Velcro or the like, which allow the sole to be removed and, if necessary, quickly replaced.

This invention can be applied in the health sector with particular reference to health equipment for immobilization, support and rehabilitation of the limbs by means of prostheses and walkers.

### BACKGROUND ART

The use is known of walkers or prostheses for patients suffering from fractures or sprains in the plantar area or in portions of the lower joint of the leg and of the ankle which presents a particularly complex bone structure.

The injury suffered often involves difficulties in walking due to the pain felt when resting the foot on the ground; in particular, if the injury is a fracture, it is necessary to immobilize the injured part to ensure subsequent self-reconstruction and knitting of the bone structure.

The solutions currently known, see for example patent EP00770368, envisage the use of a substantially boot-shaped orthopaedic prosthesis, which generally presents a front half-casing that can be adapted to the tibial area and the upper part of the foot and a rear half-casing in contact with the heel and which can be adapted to the sole of the foot and the calf.

In other cases, such as for example the solution in patent VR2003A000098 filed in the name of this applicant, the walker is articulated at the level of the ankle joint, and in fact comprises a casing with an inner concave portion with each longitudinal edge presenting a wing designed to be fixed to a corresponding hinged joint, restraining a respective upright which remains free to carry out angular movements within an adjustable range.

Both in articulated walkers and in partially or fully fixed ones, the lower and outer part of the casing in which the foot is inserted is generally provided with a sole which remains rigidly attached to the respective support surface of the walker, by means of gluing, sewing or other forms of fixing.

In other cases, especially in the classic boot-type immobilizers, an overshoe-type cover is sometimes used, involving fastening by means of laces or Velcro straps, as in typical footwear.

One drawback of these known solutions is represented by the fact that the sole, normally blocked to the part of the walker that rests on the ground, above all when used for partial or total support, picks up the dirt on the ground. This can represent a serious problem, especially when considering that these walkers must be worn round the clock, without the possibility of removing them.

This means that the dirt and dust picked up by the sole can be transferred anywhere, even in the most inappropriate places, such as on cushions or on the bed, with all the obvious negative consequences.

If, instead, the overshoe-type covering is used, the patient may encounter practical problems, mainly regarding the difficulty of fitting and removing the shoe cover with the lace or Velcro fastening means.

### DESCRIPTION OF THE INVENTION

This invention proposes to provide a sole that can be fitted to a plantar walker for the foot and/or the ankle in a removable way, in order to eliminate or significantly reduce the problems indicated above.

This invention also proposes to provide a removable sole for plantar walkers which is easy to produce as well as being reliable and safe.

This is achieved by means of a sole for plantar walkers, for the foot and/or the ankle, with the features described in the main claim.

The dependent claims described advantageous embodiments of the invention.

The removable sole according to the invention, which can be fitted on plantar walkers, is substantially made as a single piece or in several parts or layers according to the required consistency and adherence to the ground. Its upper part, the part opposite the walking surface, includes a layer of non-permanent adhesive material, such as Velcro or the like, the other part of which is applied to the lower walking surface of the walker casing.

The walker and the respective casing are, for example, obtained by moulding rigid and non-allergic composite plastic material and/or lightweight metal alloy and the gripping part of the non-permanent adhesive material or Velcro, provided with small hooks, can be fitted on either the casing of the walker or on the sole.

### DESCRIPTION OF THE DRAWINGS

Other features and advantages of the invention will become evident on reading the following description of one embodiment of the invention, given as a non-binding example, together with the help of the accompanying drawings in which:
- figure 1 shows a prospective side view of a walker according to the invention, to which a sole has been fitted by means of temporary fixing means made from bi-adhesive material such as Velcro or the like;
- figure 2 represents a detailed view of figure 1 showing the sole in a detached position with respect to the lower part of the casing of the walker;
- figure 3 is an enlarged view of a detail of the two joining surfaces of the sole and the casing;
- figure 4 shows a schematic view of the sole during removal from the casing of the walker.

### DESCRIPTION OF ONE EMBODIMENT

In the figures, the reference number 10 indicates in general a plantar walker, in the case in question a plantar walker for the foot and/or the ankle.

The walker 10 comprises a casing 11 with its inner concave portion being designed to accommodate the patient's foot.

In the example shown in the drawings the front part of the casing 11 is quite low, gradually increasing in height towards the central part and the rear portion 12 of the walker 10.

Close to the rear part 12, each side of the casing 11 presents a wing 13 to which a corresponding upright 14 can be attached.

A hinge or other means of release can be fitted between each wing 13 and each upright 14, while other parts of the casing 11 can be equipped with fixed or hinged slots 15 to accommodate straps for fixing the walker 10 to the user's foot.

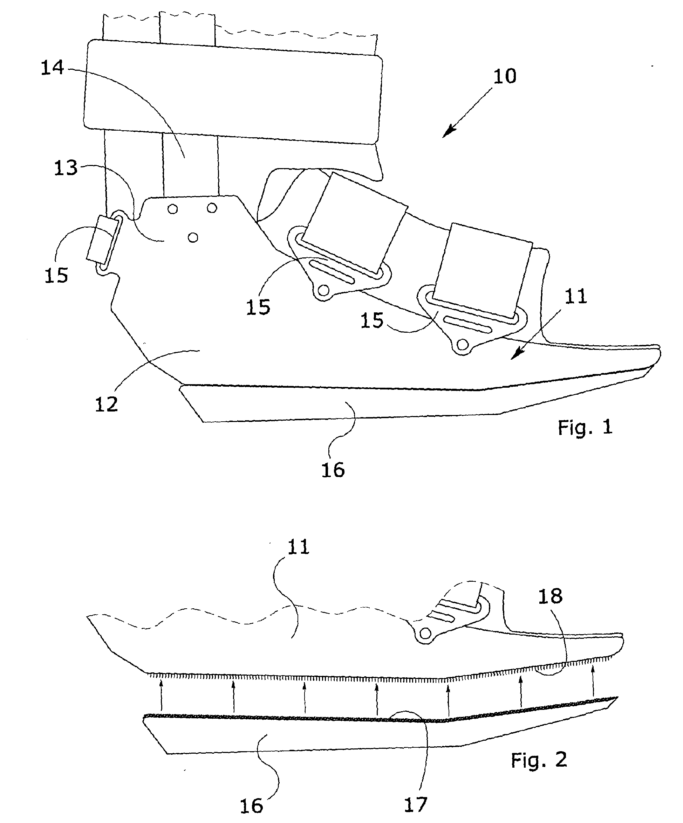

As can be seen in figures 1 and 2, the particular feature of the invention is the presence of the sole 16, and the fact that the sole 16 is the removable type, being fixed to the casing by means of non-permanent bi-adhesive material.

In fact the upper part of the sole 16 opposite the walking surface and the lower part of the casing 11 are equipped with layers of non-permanent bi-adhesive material, such as Velcro or the like, that is to say one layer 17 on the sole and another layer 18 on the casing.

The sole 16 can be made in a single piece, for example by means of injection or thermofusion of plastic material, or in several parts or layers depending on the required consistency and adherence to the ground, if for example a more or less corrugated walking surface is required.

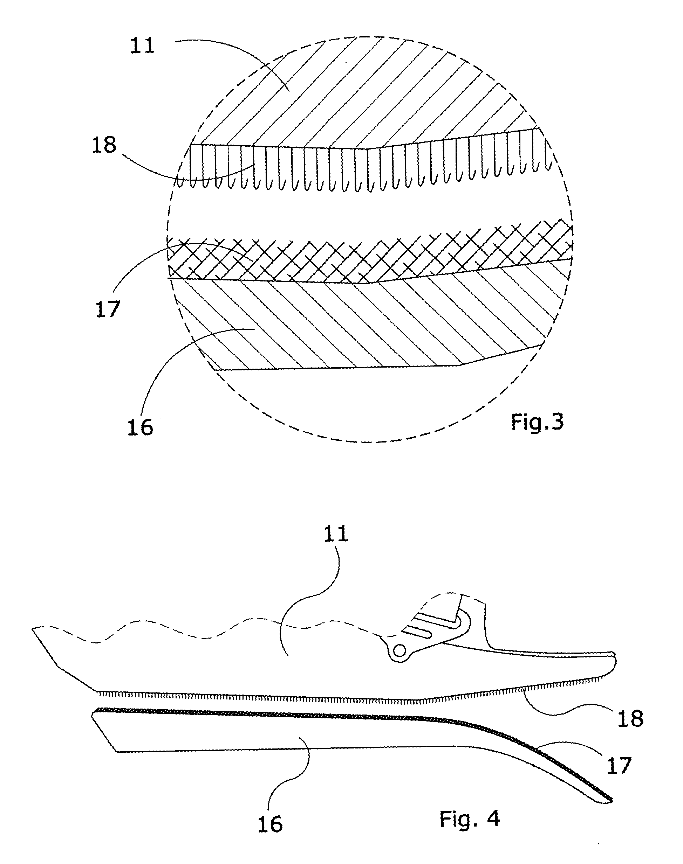

In the detail shown in figure 3 it can be seen that the layer 17 fitted on the sole is the honeycomb type, while the adhesive layer 18 on the casing is the hooked type.

In the same way and achieving the same results, the hooked material can be fitted on the sole and the honeycomb layer on the casing.

According to a particularly advantageous embodiment of the invention, the layer of non-permanent bi-adhesive material can be made in a single piece, that is covering all or part of the surface of the sole and the casing.

According to other particularly advantageous embodiments of the invention, the layer of non-permanent bi-adhesive material can be made in strips or sectors, depending on the required degree of adherence.

To use the solution according to the invention it is therefore sufficient to bring the upper surface of the sole into contact with the lower surface of the casing and exert a slight pressure to ensure the complete reciprocal adherence of the layers of bi-adhesive material 17 and 18.

When it is necessary to use the walker "at rest" for example resting the foot on a cushion or on a bed, it is sufficient to remove the sole 16 simply gripping one end and pulling until it is completely detached from the casing.

As can be seen, the described solution offers significant practical advantages due to the ease with which the sole can be fitted to or removed from the orthopaedic device, especially considering the difficulty in movement of a patient fitted with a walker on one or in some cases on both legs.

It is also possible to envisage easy replacement of the sole when it is worn out, or when a different corrugation is required.

The invention is described above with reference to a preferred embodiment.

The invention is nevertheless susceptible to numerous variations which lie within the framework of technical equivalents.

## Claims

1. A sole (16) which can be applied in particular to plantar type walkers (10) for the foot and/or the ankle or for the lower part of the leg, in which the walker presents a casing (11) in which the foot is inserted, **characterised in that** the sole (16) is the removable type and **in that** the casing of the walker can be fixed to the removable sole (16) by means of non-permanent bi-adhesive material.

2. A sole which can be applied to walkers (10) according to the foregoing claim, **characterised in that** the upper part of the sole (16) opposite the walking surface and the lower part of the casing (11) are fitted with layers of non-permanent bi-adhesive material, such as Velcro or the like, with one layer (17) on the sole and one layer (18) on the casing.

3. A sole which can be applied to walkers (10) according to either of the foregoing claims, **characterised in that** the layer (17) fitted on the sole is the honeycomb type, while the adhesive layer (18) fitted on the casing is the hooked type.

4. A sole which can be applied to walkers (10) according to any of the foregoing claims, **characterised in that** to obtain the same results the hooked material (18) can be indifferently applied on the sole (16) and the honeycomb material (17) on the casing (11).

5. A sole which can be applied to walkers (10) according to any of the foregoing claims, **characterised in that** the layer of non-permanent bi-adhesive material is made in a single piece, covering all or part of the surface of the sole and the casing in a single piece.

6. A sole which can be applied to walkers (10) according to any of the foregoing claims, **characterised in that** the layer of non-permanent bi-adhesive material can be made in strips or sectors, depending the type of adherence required.
